(19) Europäisches Patentamt
European Patent Office
Office européen des brevets

(11) Veröffentlichungsnummer: **0 410 172 A2**

(12) # EUROPÄISCHE PATENTANMELDUNG

(21) Anmeldenummer: **90112825.6**

(22) Anmeldetag: **05.07.90**

(51) Int. Cl.⁵: **C07C 50/32**, C07C 255/54, C07C 323/20, A01N 31/16, A01N 37/40

(30) Priorität: **18.07.89 DE 3923657**

(43) Veröffentlichungstag der Anmeldung: **30.01.91 Patentblatt 91/05**

(84) Benannte Vertragsstaaten: **BE CH DE ES FR GB IT LI NL**

(71) Anmelder: **BAYER AG**

**D-5090 Leverkusen 1 Bayerwerk(DE)**

(72) Erfinder: **Lindner, Werner, Dr.**
**Maerchenstrasse 39**
**D-5000 Köln 80(DE)**
Erfinder: **Brandes, Wilhelm, Dr.**
**Eichendorffstrasse 3**
**D-5653 Leichlingen(DE)**
Erfinder: **Benedikt, Becker, Dr., z.Zt. Bayer Italia**
**Versuchstation für Pflanzenschutz**
**I-3950 Pineta di Laives, Bozen(IT)**
Erfinder: **Erdelen, Christoph, Dr.**
**Unterbüscherhof 22**
**D-5653 Leichlingen(DE)**

(54) **2-Halogen-3-phenoxy-1,4-naphthochinon-Derivate.**

(57) Neue 2-Halogen-3-phenoxy-1,4-naphthochinon-Derivate der Formel (I),

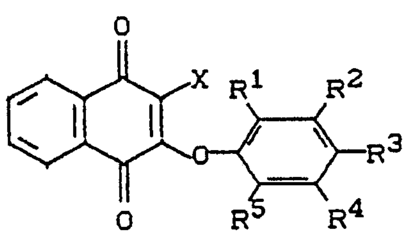

(I)

in welcher
$R^1$-$R^5$ und X die in der Beschreibung angegebene Bedeutung haben, ihre Verwendung zur Bekämpfung von Schädlingen und neue Zwischenprodukte.

Die neuen Verbindungen der Formel (I) können nach Analogieverfahren hergestellt werden, z.B. indem man geeignete Phenole mit 2,3-Dihalogennaphthochinonen umsetzt oder geeignete 2-Phenoxy-1,4-naphthochinone halogeniert. Die 2-Phenoxy-1,4-naphthochinone sind auch neu und durch die Formel (IV) dargestellt und können auch nach Analogieverfahren hergestellt werden, z.B. aus geeigneten Phenolen und 2-Halogen-1,4-naphthochinon.

EP 0 410 172 A2

Xerox Copy Centre

# 2-HALOGEN-3-PHENOXY-1,4-NAPHTHOCHINON-DERIVATE

Die Erfindung betrifft neue 2-Halogen-3-phenoxy-1,4-naphthochinon-Derivate, mehrere Verfahren zu ihrer Herstellung und ihre Verwendung als Schädlingsbekämpfungsmittel insbesondere als Fungizide und Akarizide.

Es ist bekannt, daß bestimmte 1,4-Naphthochinon-Derivate, beispielsweise 2-Chlor-3-(4-methoxyphenoxy)-1,4-naphthochinon, 2-Chlor-3-(4-chlorphenoxy)-1,4-naphthochinon, 2-Chlor-3-(4-methylphenoxy)-1,4-naphthochinon, 2-Chlor-3-(4-nitrophenoxy)-1,4-naphthochinon oder 2-Brom-3-phenoxy-1,4-naphthochinon, fungizide Eigenschaften besitzen (vgl. Pestic. Sci. 15 , 235 - 240 (1984)).

Die fungizide und akarizide Wirksamkeit dieser vorbekannten Verbindungen ist jedoch nicht in allen Anwendungsgebieten völlig zufriedenstellend.

Weiterhin ist das 2-Chlor-3-(2,5-dimethylphenoxy)-1,4-naphthochinon bekannt (vgl. CA 99 (5) : 38 149 n; Zh. Org. Khim., 19 (1), 158-64 (1983)). Über die physiologische Wirksamkeit dieser Verbindung ist jedoch nichts bekannt.

Es wurden neue 2-Halogen-3-phenoxy-1,4-naphthochinonDerivate der allgemeinen Formel (I),

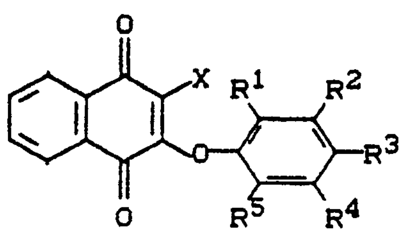

(I)

in welcher

$R^1$-$R^5$ unabhängig voneinander für Wasserstoff, Halogen, Cyano, Nitro, Alkyl, Halogenalkyl, Alkoxy, Halogenalkoxy, Alkylthio, Alkylsulfonyl, Alkylsulfinyl, Halogenalkylthio, Aryloxy, Arylthio, Halogenalkylsulfinyl oder Halogenalkylsulfonyl und

X für Halogen steht,

ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen und X jeweils für Halogen steht,

gefunden.

Weiterhin wurde gefunden, daß man die neuen 2-Halogen-3-phenoxy-1,4-naphthochinon-Derivate der allgemeinen Formel (I)

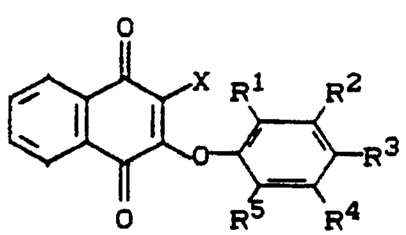

(I)

in welcher

$R^1$-$R^5$ unabhängig voneinander für Wasserstoff, Halogen, Cyano, Nitro, Alkyl, Halogenalkyl, Alkoxy, Halogenalkoxy, Alkylthio, Alkylsulfonyl, Alkylsulfinyl, Halogenalkylthio, Aryloxy, Arylthio, Halogenalkylsulfinyl oder Halogenalkylsulfonyl und

X für Halogen steht,

ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen und X jeweils für Halogen steht,

erhält, wenn man

(a) Phenol-Derivate der allgemeinen Formel (II)

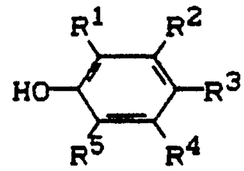

(II)

in welcher
$R^1$-$R^5$ die oben angegebene Bedeutung haben, ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen,
mit 2,3-Dihalogen-1,4-naphthochinonen der allgemeinen Formel (III)

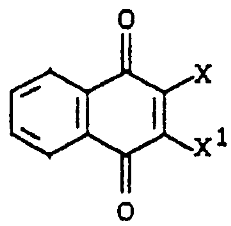

(III)

in welcher
X die oben angegebene Bedeutung hat und
$X^1$ für Halogen steht,
gegebenenfalls in Gegenwart eines Verdünnungsmittels und gegebenenfalls in Gegenwart eines basischen Reaktionshilfsmittels umsetzt, oder
(b) 2-Phenoxy-1,4-naphthochinon-Derivate der allgemeinen Formel (IV)

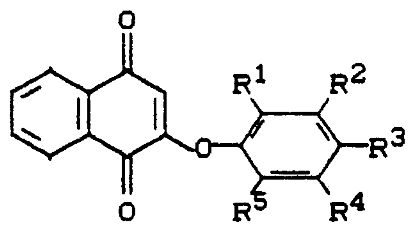

(IV)

in welcher
$R^1$-$R^5$ die oben angegebene Bedeutung haben,
ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen,
mit Halogenen ($X_2$) gegebenenfalls in Gegenwart eines Verdünnungsmittels umsetzt und anschließend gegebenenfalls in Gegenwart eines Verdünnungsmittels und gegebenenfalls in Gegenwart eines basischen Reaktionshilfsmittels Halogenwasserstoff (H-X) eliminiert.

Schließlich wurde gefunden, daß die neuen 2-Halogen-3-phenoxy-1,4-naphthochinon-Derivate der allgemeinen Formel (I) sehr gute Wirkung gegen Schädlinge zeigen.

Überraschenderweise zeigen die erfindungsgemäßen 2-Halogen-3-phenoxy-1,4-naphthochinon-Derivate der allgemeinen Formel (I), ausgenommen die ausgeschlossenen Verbindungen, erheblich höhere fungizide und akarizide Wirksam keit als die aus dem Stand der Technik bekannten 1,4-Naphthochinon-Derivate, wie beispielsweise das 2-Chlor-3-(4-methylphenoxy)-1,4-naphthochinon oder das 2-Chlor-3-(4-methoxyphenoxy)-1,4-naphthochinon, welche chemisch und wirkungsmäßig naheliegende Verbindungen sind.

Die erfindungsgemäßen 2-Halogen-3-phenoxy-1,4-naphthochinon-Derivate sind durch die Formel (I) allgemein definiert. Der Phenoxyring in Verbindungen der Formel (I) kann 1 bis 5-fach, gleich oder verschieden substituiert sein, bevorzugt ist die ein- bis dreifache und besonders bevorzugt die ein- oder zweifache Substitution.

Bevorzugt sind Verbindungen der Formel (I), bei welchen
$R^1$-$R^5$ unabhängig voneinander für Halogen oder geradkettiges oder verzweigtes Alkyl mit 1 bis 3 Kohlenstoffatomen steht
oder
$R^1$-$R^5$ für eine Kombination aus Wasserstoff und 1 bis 3, gleichen oder verschiedenen der folgenden Substituenten steht:
Halogen, Cyano, Nitro, geradkettiges oder verzweigtes Alkyl mit 1 bis 4 Kohlenstoffatomen, geradkettiges oder verzweigtes Halogenalkyl mit 1 bis 3 Kohlenstoffatomen und 1 bis 7 gleichen oder verschiedenen Halogenatomen, geradkettiges oder verzweigtes Alkoxy oder Halogenalkoxy mit jeweils 1 bis 3 Kohlenstoffatomen und gegebenenfalls 1 bis 7 gleichen oder verschiedenen Halogenatomen, geradkettiges oder verzweigtes Alkylthio, Halogenalkylthio oder Halogenalkylsulfonyl mit jeweils 1 bis 3 Kohlenstoffatomen und gegebenenfalls 1 bis 7 gleichen oder verschiedenen Halogenatomen, Phenoxy oder Phenylthio, wobei die Reihenfolge der Substituenten beliebig ist,
und
X für Halogen steht,
ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen und X jeweils für Halogen steht.

Besonders bevorzugt sind Verbindungen der Formel (I), bei welchen
$R^1$-$R^5$ unabhängig voneinander für Fluor, Chlor, Brom, Methyl oder Ethyl stehen
oder
$R^1$-$R^5$ für eine Kombination aus Wasserstoff und 1 oder 2, gleichen oder verschiedenen der folgenden Substituenten steht:
Fluor, Chlor, Brom, Cyano, Nitro, Methyl, Ethyl, n- oder i-Propyl, Trifluormethyl, 2,2,2-Trifluorethyl, Methoxy, Ethoxy, Trifluormethoxy, 2,2,2-Trifluorethoxy, Methylthio, Ethylthio, Trifluormethylthio oder 2,2,2-Trifluorethylthio, wobei die Reihenfolge der Substituenten beliebig ist
und
X für Fluor, Chlor oder Brom steht,
ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen und x jeweils für Halogen steht.

Verwendet man beispielsweise 2,3-Dichlor-1,4-naphthochinon und 3-Fluorphenol als Ausgangsstoffe, so läßt sich der Reaktionsablauf des erfindungsgemäßen Verfahrens (a) durch das folgende Formelschema darstellen:

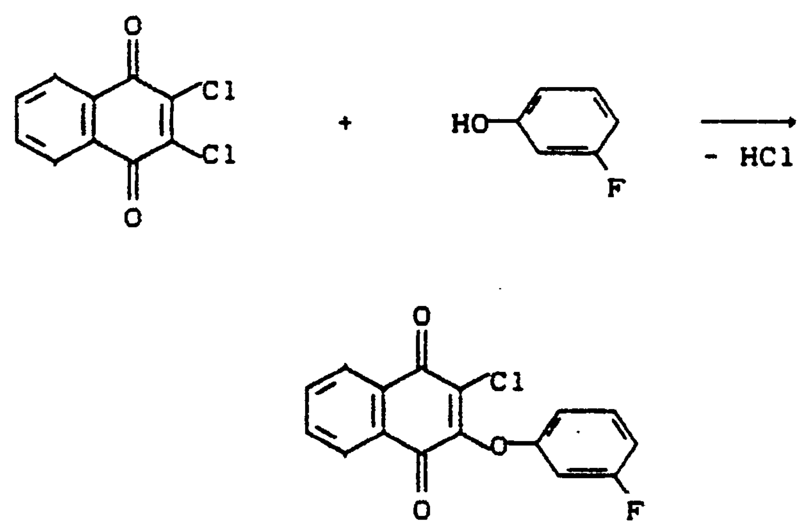


Verwendet man beispielsweise 2-(3-Fluorphenoxy)-1,4-naphthochinon und Chlor als Ausgangsstoffe, so läßt sich der Reaktionsablauf des erfindungsgemäßen Verfahrens (b) durch das folgende Formelschema darstellen:

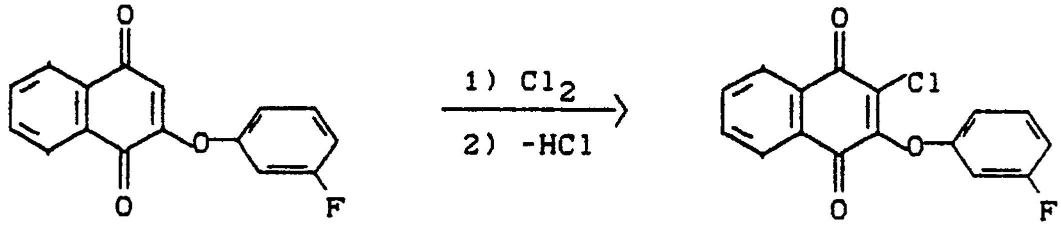


Die beim erfindungsgemäßen Verfahren (a) zur Herstellung von Verbindungen der Formel (I) als Ausgangsstoffe zu verwendenden 2,3-Dihalogen-1,4-naphthochinon-Derivate sind durch die Formel (III) allgemein definiert.

In Formel (III) hat X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für X angegeben wurden.

$X^1$ steht für Halogen, insbesondere für Fluor, Chlor oder Brom.

2,3-Dihalogen-1,4-naphthochinon-Derivate der Formel (III) sind allgemein bekannte Verbindungen der organischen Chemie (vergl. z.B. Horst, W.P. et al., Ind. Eng. Chem. 35, 1255, 1943).

Die beim erfindungsgemäßen Verfahren (a) zur Herstellung von Verbindungen der Formel (I) als Ausgangsstoffe zu verwendenden Phenol-Derivate sind durch die Formel (II) allgemein definiert.

In Formel (II) haben $R^1$, $R^2$, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden.

Phenol-Derivate der Formel (II) sind allgemein bekannte Verbindungen der organischen Chemie und können nach allgemein bekannten Verfahren hergestellt werden (vgl. z.B. Beilstein, "Handbuch der organischen Chemie", Bd. VI, 4. Ergänzungswerk, z.B. S. 770, 810, 1940 u.a.).

Die beim erfindungsgemäßen Verfahren (b) zur Herstellung von Verbindungen der Formel (I) als Ausgangsstoffe zu verwendenden 2-Phenoxy-1,4-naphthochinon-Derivate sind durch die Formel (IV) allgemein definiert.

In Formel (IV) haben $R^1$, $R^2$, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden.

Die 2-Phenoxy-1,4-naphthochinon-Derivate der Formel (IV) sind neu. Man erhält die neuen Verbindungen der Formel (IV),

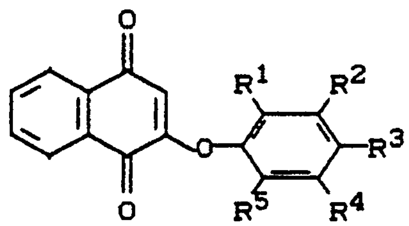

(IV)

in welcher

$R^1$, $R^2$, $R^3$, $R^4$ und $R^5$ die oben angegebene Bedeutung haben, wenn man Phenol-Derivate der Formel (II)

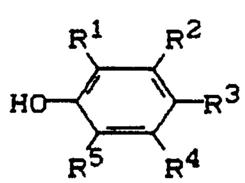

(II)

in welcher

$R^1$, $R^2$, $R^3$, $R^4$ und $R^5$ die oben angegebene Bedeutung haben, mit 2-Halogen-1,4-naphthochinon der allgemeinen Formel (V)

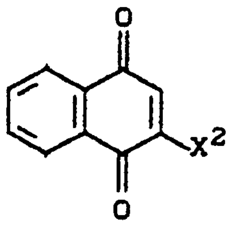

(V)

in welcher
$X^2$ für Halogen, insbesondere für Chlor oder Brom steht,
gegebenenfalls in Gegenwart eines Verdünnungsmittels wie beispielsweise Benzol oder Toluol und gegebenenfalls in Gegenwart einer Base wie Kaliumcarbonat oder Natriumcarbonat bei Temperaturen zwischen 0° C und 90° C umsetzt.

Die 2-Halogen-1,4-naphthochinone der Formel (V) sind ebenso wie die Phenol-Derivate der Formel (II) allgemein bekannte Verbindungen der organischen Chemie und nach allgemein bekannten Verfahren herstellbar.

Als Verdünnungsmittel zur Durchführung des erfindungsgemäßen Verfahrens (a) kommen vorzugsweise inerte organische Lösungsmittel infrage.

Hierzu gehören insbesondere aliphatische, alicyclische oder aromatische, gegebenenfalls halogenierte Kohlenwasserstoffe, wie beispielsweise Benzin, Ligroin, Benzol, Toluol, Xylol, Chlorbenzol, Petrolether, Pentan, Hexan, Heptan, Cyclohexan, Dichlormethan, Chloroform, Tetrachlorkohlenstoff; Ether, wie Diethylether, Dioxan, Tetrahydrofuran oder Ethylenglykoldimethyl oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester oder Basen, wie Pyridin.

Das erfindungsgemäße Verfahren (a) wird gegebenenfalls in Gegenwart eines basischen Reaktionshilfsmittels durchgeführt.

Als solche kommen alle üblichen anorganischen oder organischen Basen in Frage, wie beispielsweise Alkalihydroxide, Alkalicarbonate, tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU). Vorzugsweise verwendet man Alkalicarbonate, wie beispielsweise Natriumcarbonat oder Kaliumcarbonat.

Die Reaktionstemperaturen können bei der Durchführung des erfindungsgemäßen Verfahrens (a) in einem größeren Bereich variiert werden. Im allgemeinen arbeitet man bei Temperaturen zwischen + 50° C und + 160° C, vorzugsweise bei Temperaturen zwischen + 60° C und + 90° C.

Das erfindungsgemäße Verfahren (a) wird üblicherweise unter Normaldruck durchgeführt. Es ist jedoch auch möglich, unter erhöhtem Druck zu arbeiten.

Zur Durchführung des erfindungsgemäßen Verfahrens (a) setzt man pro Mol an 2,3-Dihalogennaphthochinon der Formel (III) 1 bis 5 Mol, vorzugsweise 1 bis 2 Mol an Phenol-Derivat der Formel (II) und gegebenenfalls 0,5 bis 1 Mol an basischem Reaktionshilfsmittel ein. Die Reaktionsdurchführung, Aufarbeitung und Isolierung der Reaktionsprodukte erfolgt in Analogie zu allgemein bekannten Verfahren.

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) genannten Verdünnungsmittel, es können jedoch auch organische Säuren wie z. B. Essigsäure verwendet werden.

Das erfindungsgemäße Verfahren (b) wird gegebenenfalls in Gegenwart eines basischen Reaktionshilfsmittels durchgeführt.

Als solche kommen alle üblichen anorganischen oder organischen Basen in Frage, wie beispielsweise Alkali- und Erdalkalihydroxide, Alkalicarbonate, Alkaliacetate, tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU). Vorzugsweise verwendet man Alkaliacetate, wie beispielsweise Natriumacetat.

Die Reaktionstemperaturen können bei der Durchführung des erfindungsgemäßen Verfahrens (b) in einem größeren Bereich variiert werden. Im allgemeinen arbeitet man bei Temperaturen zwischen + 80° C und + 140° C, vorzugsweise bei Temperaturen zwischen + 100° C und + 120° C.

Das erfindungsgemäße Verfahren (b) wird üblicherweise unter Normaldruck durchgeführt. Es ist jedoch auch möglich, unter erhöhtem Druck zu arbeiten.

Zur Durchführung des erfindungsgemäßen Verfahrens (b) setzt man pro Mol an 2-Phenoxy-1,4-naphthochinon-Derivat der Formel (IV) 1 bis 5 Mol, vorzugsweise 1 bis 2 Mol an Halogen und gegebenenfalls 1 bis 2 Mol an basischem Reaktionshilfsmittel ein. Die Reaktionsdurchführung erfolgt in Analogie zu

allgemein bekannten Verfahren.

Die erfindungsgemäßen Wirkstoffe weisen eine stark mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe zeigen insbesondere eine starke fungizide Wirkung, z. B. gegen den Erreger von Fäule an Tomaten (Phytophthora), eine protektive Wirkung gegen den Apfelschorferreger (Venturia inaequalis), außerdem gegen Plasmopara an Reben, gegen Botrytis- und Sclerotiniapilze sowie gegen Pyricularia und Pellicularia an Reis.

Die Wirkstoffe eignen sich außerdem zur Bekämpfung von tierischen Schädlingen, vorzugsweise Arthropoden und Nematoden, insbesondere Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis,
Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anaplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Cheuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe zeigen insbesondere hervorragende akarizide Wirkung, auch gegen resistente Spinnmilben, wie z. B. Tetranychus urticae, welche Nutzpflanzen, wie z. B. Bohnen schädigen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als

Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Ge steine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die biologische Wirksamkeit der erfindungsgemäßen Verbindungen wird anhand der Anwendungsbeispiele erläutert.

## Herstellungsbeispiele

### Beispiel 1

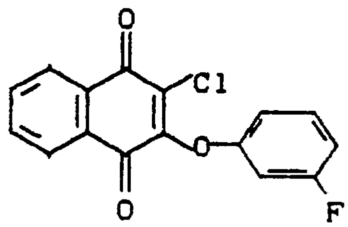

(Verfahren (a))

6,7 g (0,06 Mol) 3-Fluorphenol und 3,8 g (0,03 Mol) Kaliumcarbonat werden in Benzol 3 Stunden lang am Wasserabscheider zum Sieden erhitzt. Zu diesem Reaktionsgemisch tropft man anschließend 11,3 g (0,05 Mol) 2,3-Dichlor-1,4-naphthochinon, gelöst in Tetrahydrofuran, und erhitzt 15 Stunden am Rückfluß. Nach dem Abkühlen verdünnt man mit Wasser und filtriert das Reaktionsprodukt ab. Man erhält 10,5 g (69 %. der Theorie) 2-Chlor-3-(3-fluorphenoxy)-1,4-naphthochinon vom Schmelzpunkt 123° C.

(Verfahren (b))

5 g (0,019 Mol) 2-(3-Fluorphenoxy)-1,4-naphthochinon werden in 100 ml Eisessig gelöst. Bei Raumtemperatur leitet man 3 g (0,043 Mol) Chlorgas ein und rührt weitere 12 Stunden bei Raumtemperatur. Der gebildete Fest stoff wird abfiltriert, in 100 ml Eisessig suspendiert und nach Zugabe von 3 g (0,036 Mol) Natriumacetat für 20 Minuten am Rückfluß erhitzt.

Anschließend wird das Reaktionsgemisch auf Wasser gegossen und der ausgefallene Feststoff abfiltriert. Man erhält 4,3 g (76 % der Theorie) 2-Chlor-3-(3-fluorphenoxy)-1,4-naphthochinon vom Schmelzpunkt 123° C.

In entsprechender Weise, analog zu den oben beschriebenen Verfahren (a) und (b) und gemäß den allgemeinen Angaben zur Herstellung, erhält man die folgenden 2-Halogen-3-phenoxy-1,4-naphthochinon-Derivate der allgemeinen Formel (I):

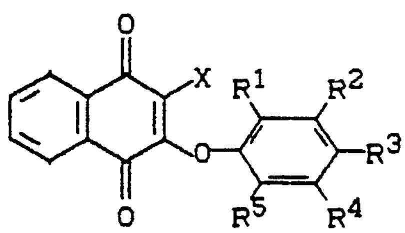

(I)

Tabelle 1

| Bsp. Nr. | $R^1$ | $R^2$ | $R^3$ | $R^4$ | $R^5$ | phys. Daten Fp. |
|---|---|---|---|---|---|---|
| 2 | H | Cl | H | H | H | 134° C |
| 3 | H | H | H | H | Cl | 146° C |
| 4 | H | H | H | H | F | 136° C |
| 5 | F | H | F | H | H | 164° C |
| 6 | H | H | F | H | H | 139° C |
| 7 | H | H | $-OCF_3$ | H | H | 104° C |
| 8 | H | H | $-SCF_3$ | H | H | 107° C |
| 9 | $-CH_3$ | H | H | H | H | 135° C |
| 10 | Cl | H | Cl | H | H | 151° C |
| 11 | Cl | H | $-CF_3$ | H | H | 189° C |
| 12 | H | $-CF_3$ | H | $-CF_3$ | H | 158° C |
| 13 | H | H | H | $-OCH_3$ | H | 102° C |
| 14 | H | H | H | H | $-OCH_3$ | 141° C |
| 15 | H | H | Cl | Cl | H | 129° C |
| 16 | H | H | -CN | H | H | 169° C |
| 17 | H | H | $-OCF_3$ | H | Cl | 140° C |
| In den Beispielen 2 - 17 steht x für Chlor | | | | | | |

Herstellung der Ausgangsverbindungen

Beispiel (IV)-1

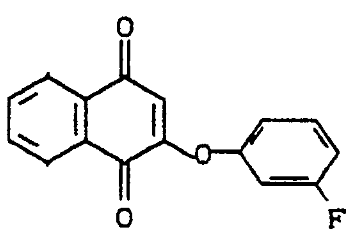

3,6 g (0,03 Mol) 3-Fluorphenol werden mit 2,2 g (0,016 Mol) Kaliumcarbonat in 50 ml Benzol für 3 Stunden am Wasserabscheider gekocht. Anschließend werden 5,4 g (0,028 Mol) 2-Chlor-1,4-naphthochinon, gelöst in Tetrahydrofuran, zugetropft und die Mischung 15 Stunden am Rückfluß gekocht. Nach Zugabe von Wasser wird das ausgefallene Reaktionsprodukt abgesaugt.
Man erhält 5,9 g (78 % der Theorie) 2-(3-Fluorphenoxy)-1,4-naphthochinon vom Schmelzpunkt 109° C.

Anwendungsbeispiele

In den folgenden Anwendungsbeispielen wurden die nachstehend aufgeführten Verbindungen als Vergleichssubstanzen eingesetzt:

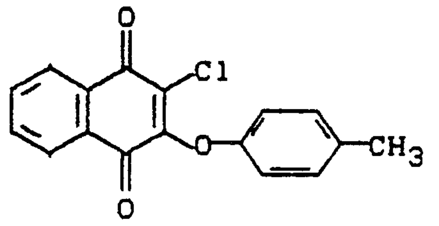

(A)

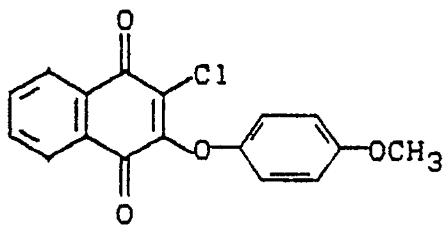

(B)

(beide bekannt aus Pestic. Sci. 15 , 235 - 240 (1984)).

Beispiel A

Phytophthora-Test (Tomate) / protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Eine deutliche Überlegenheit in der Wirksamkeit bei einer Wirkstoffkonzentration von 10 ppm gegenüber dem Stand der Technik zeigen in diesem Test z. B. die Verbindungen gemäß den Herstellungsbeispielen: 1, 4, 5, 6, 7 und 9.

Beispiel B

Venturia-Test (Apfel) / protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 ° C und einer relativen Luftfeuchtigkeit von ca. 70 % aufgestellt.

12 Tage nach der Inokulation erfolgt die Auswertung.

Eine deutliche Überlegenheit in der Wirksamkeit bei einer Wirkstoffkonzentration von 5 ppm gegenüber dem Stand der Technik zeigen in diesem Test z. B. die Verbindungen gemäß den Herstellungsbeispielen: 1, 4, 5, 6, 7, 9 und 10.

Beispiel C

Tetranychus-Test (resistent)

Lösungsmittel: 7 Gewichtsteile Dimethylformamid
Emulgator: 1 Gewichtsteile Alkylarylpolyglykoläther

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z. B. die folgenden Verbindungen der Herstellungsbeispiele 2, 3 und 7 überlegene Wirksamkeit gegenüber dem Stand der Technik bei einer Wirkstoffkonzentration von 0,1 %.

**Ansprüche**

1. 2-Halogen-3-phenoxy-1,4-naphthochinon-Derivate der allgemeinen Formel (I),

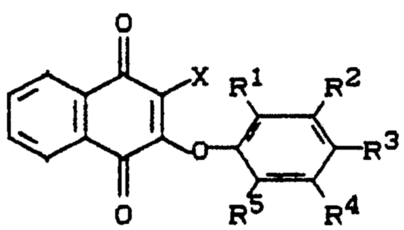

(I)

in welcher
$R^1$-$R^5$ unabhängig voneinander für Wasserstoff, Halogen, Cyano, Nitro, Alkyl, Halogenalkyl, Alkoxy, Halogenalkoxy, Alkylthio, Alkylsulfonyl, Alkylsulfinyl, Halogenalkylthio, Aryloxy, Arylthio, Halogenalkylsulfinyl oder Halogenalkylsulfonyl und
X für Halogen steht,
ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen und X jeweils für Halogen steht.

2. 2-Halogen-3-phenoxy-1,4-naphthochinon-Derivate der Formel (I) gemäß Anspruch 1, in welcher
$R^1$-$R^5$ unabhängig voneinander für Halogen oder geradkettiges oder verzweigtes Alkyl mit 1 bis 3 Kohlenstoffatomen steht
oder
$R^1$-$R^5$ für eine Kombination aus Wasserstoff und 1 bis 3, gleichen oder verschiedenen der folgenden Substituenten steht:
Halogen, Cyano, Nitro, geradkettiges oder verzweigtes Alkyl mit 1 bis 4 Kohlenstoffatomen, geradkettiges oder verzweigtes Halogenalkyl mit 1 bis 3 Kohlenstoffatomen und 1 bis 7 gleichen oder verschiedenen Halogenatomen, geradkettiges oder verzweigtes Alkoxy oder Halogenalkoxy mit jeweils 1 bis 3 Kohlenstoffatomen und gegebenenfalls 1 bis 7 gleichen oder verschiedenen Halogenatomen, geradkettiges oder

verzweigtes Alkylthio, Halogenalkylthio oder Halogenalkylsulfonyl mit jeweils 1 bis 3 Kohlenstoffatomen und gegebenenfalls 1 bis 7 gleichen oder verschiedenen Halogenatomen, Phenoxy oder Phenylthio und
X für Halogen steht,
ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen und X jeweils für Halogen steht.

3. 2-Halogen-3-phenoxy-1,4-naphthochinon-Derivate der Formel (I) gemäß Anspruch 1, in welcher
$R^1$-$R^5$ unabhängig voneinander für Fluor, Chlor, Brom, Methyl oder Ethyl stehen,
oder
$R^1$-$R^5$ für eine Kombination aus Wasserstoff und 1 oder 2, gleichen oder verschiedenen der folgenden Substituenten steht:
Fluor, Chlor, Brom, Cyano, Nitro, Methyl, Ethyl, n- oder i-Propyl, Trifluormethyl, 2,2,2-Trifluorethyl, Methoxy, Ethoxy, Trifluormethoxy, 2,2,2-Trifluorethoxy, Methylthio, Ethylthio, Trifluormethylthio oder 2,2,2-Trifluorethylthio
und
X für Fluor, Chlor oder Brom steht,
ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen und X jeweils für Halogen steht.

4. Verfahren zur Herstellung von 2-Halogen-3-phenoxy-1,4-naphthochinon-Derivaten der allgemeinen Formel (I)

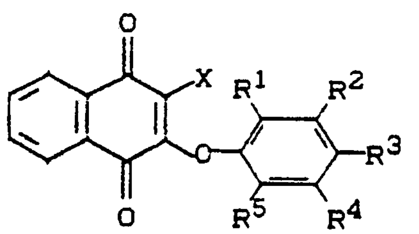

(I)

in welcher
$R^1$-$R^5$ unabhängig voneinander für Wasserstoff, Halogen, Cyano, Nitro, Alkyl, Halogenalkyl, Alkoxy, Halogenalkoxy, Alkylthio, Alkylsulfonyl, Alkylsulfinyl, Halogenalkylthio, Aryloxy, Arylthio, Halogenalkylsulfinyl oder Halogenalkylsulfonyl und
X für Halogen steht,
ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen und X jeweils für Halogen steht,
dadurch gekennzeichnet, daß man

(a) Phenol-Derivate der allgemeinen Formel (II)

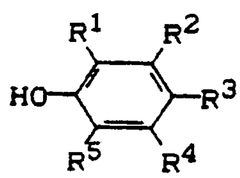

(II)

in welcher
$R^1$-$R^5$ die oben angegebene Bedeutung haben, ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen,
mit 2,3-Dihalogen-1,4-naphthochinonen der allgemeinen Formel

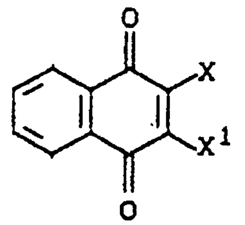

(III)

in welcher
X die oben angegebene Bedeutung hat und
$X^1$ für Halogen steht,
gegebenenfalls in Gegenwart eines Verdünnungsmittels und gegebenenfalls in Gegenwart eines basischen Reaktionshilfsmittels umsetzt, oder
(b) 2-Phenoxy-1,4-naphthochinon-Derivate der allgemeinen Formel (IV)

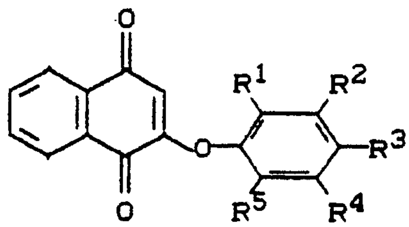

(IV)

in welcher
$R^1$-$R^5$ die oben angegebene Bedeutung haben,
ausgenommen die Verbindungen, in denen die Reste $R^1$, $R^2$, $R^4$ und $R^5$ für Wasserstoff stehen und $R^3$ für Wasserstoff, Chlor, Nitro, Methyl oder Methoxy steht und die Verbindung, in der $R^1$ und $R^5$ für Methyl und $R^2$, $R^3$ und $R^4$ für Wasserstoff stehen,
mit Halogenen ($X_2$) gegebenenfalls in Gegenwart eines Verdünnungsmittels umsetzt und anschließend gegebenenfalls in Gegenwart eines Verdünnungsmittels und gegebenenfalls in Gegenwart eines basischen Reaktionshilfsmittels Halogenwasserstoff (H-X) eliminiert.

5. Schädlingsbekämpfungsmittel, gekennzeichnet durch einen Gehalt an mindestens einem 2-Halogen-3-phenoxy-1,4-naphthochinon-Derivat der Formel (I) nach den Ansprüchen 1 und 4.

6. Verwendung von 2-Halogen-3-phenoxy-1,4-naphthochinon-Derivaten der Formel (I) nach den Ansprüchen 1 und 4 zur Bekämpfung von Schädlingen.

7. Verfahren zur Bekämpfung von Schädlingen, dadurch gekennzeichnet, daß man 2-Halogen-3-phenoxy-1,4-naphthochinon-Derivate der Formel (I) nach den Ansprüchen 1 und 4 auf Schädlinge und/oder ihren Lebensraum einwirken läßt.

8. Verfahren zur Herstellung von Schädlingsbekämpfungsmitteln, dadurch gekennzeichnet, daß man 2-Halogen-3-phenoxy-1,4-naphthochinon-Derivate der Formel (I) nach den Ansprüchen 1 und 4 mit Streckmitteln und/oder oberflächenaktiven Mitteln vermischt.

9. 3-Phenoxyl-1,4-naphthochinon-Derivate der Formel (IV),

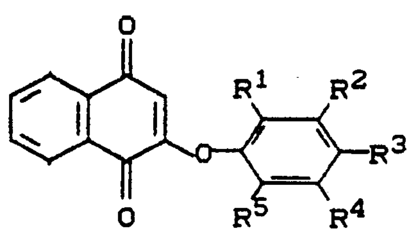

(IV)

in welcher
$R^1$-$R^5$ unabhängig voneinander für Wasserstoff, Halogen, Cyano, Nitro, Alkyl, Halogenalkyl, Alkoxy, Halogenalkoxy, Alkylthio, Alkylsulfonyl, Alkylsulfinyl, Halogenalkylthio, Aryloxy, Arylthio, Halogenalkylsulfinyl oder

Halogenalkylsulfonyl stehen

10. Verfahren zur Herstellung von 3-Phenoxy-1,4-naphthochinon-Derivaten der Formel (IV),

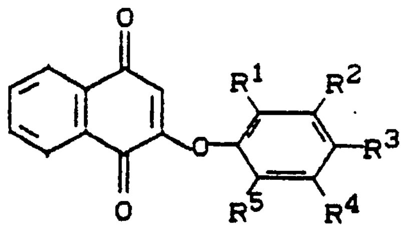

(IV)

in welcher

$R^1$-$R^5$ unabhängig voneinander für Wasserstoff, Halogen, Cyano, Nitro, Alkyl, Halogenalkyl, Alkoxy, Halogenalkoxy, Alkylthio, Alkylsulfonyl, Alkylsulfinyl, Halogenalkylthio, Aryloxy, Arylthio, Halogenalkylsulfinyl oder Halogenalkylsulfonyl stehen,

dadurch gekennzeichnet, daß man

Phenol-Derivate der Formel (II)

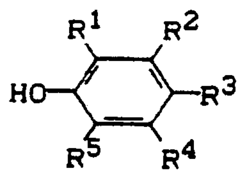

(II)

in welcher

$R^1$, $R^2$, $R^3$, $R^4$ und $R^5$ die oben angegebene Bedeutung haben, mit 2-Halogen-1,4-naphthochinon der allgemeinen Formel (V)

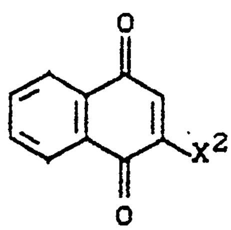

(V)

in welcher

$X^2$ für Halogen steht,

gegebenenfalls in Gegenwart eines Verdünnungsmittels und gegebenenfalls in Gegenwart einer Base bei Temperaturen zwischen 0° C und 90° C umsetzt.